(19) Europäisches Patentamt
European Patent Office
Office européen des brevets

(11) **EP 4 270 399 A1**

(12) **EUROPEAN PATENT APPLICATION**

(43) Date of publication:
**01.11.2023  Bulletin 2023/44**

(21) Application number: **22170387.9**

(22) Date of filing: **27.04.2022**

(51) International Patent Classification (IPC):
***G16B 50/00*** (2019.01)

(52) Cooperative Patent Classification (CPC):
**G16B 50/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Institut Pasteur de Dakar**
**BP 220 Dakar (SN)**

(72) Inventors:
• **BERTHET, François-Xavier**
**BP 220 Dakar (SN)**
• **SALL, Amadou Alpha**
**BP 220 Dakar (SN)**

(74) Representative: **Hoffmann Eitle**
**Hoffmann Eitle S.L.U.**
**Paseo de la Castellana 140, 3a planta**
**Edificio LIMA**
**28046 Madrid (ES)**

(54) **ENCODING GENETIC SEQUENCING INFORMATION AND USES THEREOF**

(57)    A computer-implemented method of representing a sequence of nucleotides. The method comprises the steps of: a) defining a cube or a tetrahedron using nucleotide bases, wherein each vertex of the cube or the tetrahedron is assigned a nucleotide base, wherein each of the vertices is assigned a nucleotide base such that for each vertex, the assigned base is directly connected to every other base type through an edge, b) from a selected sequence of nucleotides, the sequence of nucleotides comprising nucleotides from one of the nucleotide subsets [A,C,G,T] or [A,C,G,U], assigning the first nucleotide base of the selected sequence of nucleotides to a vertex to which the base type of the nucleotide has been assigned and sequentially assigning each subsequent nucleotide of the selected sequence of nucleotides to a corresponding vertex in the cube or the tetrahedron, such that the assigned vertex is either directly connected to the vertex of the previous nucleotide base through an edge of the cube or the tetrahedron, respectively; or in the case the nucleotide base is equal to the previous nucleotide base, the nucleotide base is assigned to the same vertex as the vertex to which the previous nucleotide base has been assigned to, and d) determining for each vertex of the cube or the tetrahedron the number of nucleotides assigned to that vertex of the cube or the tetrahedron, respectively.

Figure 3

GATC (ID)
ATCG (R1)
TCGA (R2)
CGAT (R3)
AGCT (M1)
CTAG (M2)
TAGC (M3)
GCTA (M4)

GTCA (ID)
TCAG (R1)
CAGT (R2)
AGTC (R3)
TGAC (M1)
ACTG (M2)
CTGA (M3)
GACT (M4)

GTAC (ID)
TACG (R1)
ACGT (R2)
CGTA (R3)
TGCA (M1)
CATG (M2)
ATGC (M3)
GCAT (M4)

2 x

GENOCUBE

## Description

## Technical field of the invention

The present invention pertains to the field of bioinformatics, particularly to the field of genomic information processing.

The disclosure relates to novel ways of representing a sequence of nucleotides as well as uses of these representations. More specifically, the disclosure relates to a method of representing a sequence of nucleotides in the form of a cube or a tetrahedron or in form of a matrix representing such cube or tetrahedron, to a method for tagging sequences using such representation, to a method of determining modifications into the sequence, and to a method of defining one or more markers in a nucleotide sequence, the markers characterised by a symmetry invariance.

## Background of the invention

Many fundamental laws of nature, such as the conservation of energy, the conservation of momentum, or the structure of spacetime in general relativity, emerge from the concept of symmetries (https://www.pnas.org/doi/pdf/10.1073/pnas.93.25.14256) . Symmetrical patterns were known to the Egyptians, but the mathematical structure underlying the study of symmetries, a branch of abstract algebra known as group theory, was only discovered in the nineteenth century by Abel, Galois & Lie. Since then, group theory has revolutionized the understanding of many scientific disciplines such as physics, chemistry, cryptography, information theory, etc...

The use of symbolic representations (strings of characters) to describe biological sequences has been instrumental for our understanding of genes and genomes. However, the lack of a "grammar" relating these symbols hampers a rigorous mathematical analysis of genetic information.

There is therefore a need for a better representation of biological sequences that can enable the mathematical analysis of genetic information, including the exploitation of symmetries.

## Summary of the invention

A first aspect of the invention refers to a computer-implemented method of representing a sequence of nucleotides. The method comprises the steps of:

a) defining a cube or a tetrahedron using nucleotide bases, wherein each vertex of the cube or the tetrahedron is assigned a nucleotide base, wherein each of the vertices is assigned a nucleotide base such that for each vertex, the assigned base is directly connected to every other base type through an edge,

b) from a selected sequence of nucleotides, the sequence of nucleotides comprising nucleotides from one of the nucleotide subsets [A,C,G,T] or [A,C,G, U], assigning the first nucleotide base of the selected sequence of nucleotides to a vertex to which the base type of the nucleotide has been assigned and sequentially assigning each subsequent nucleotide of the selected sequence of nucleotides to a corresponding vertex in the cube or the tetrahedron, such that the assigned vertex is either directly connected to the vertex of the previous nucleotide base through an edge of the cube or the tetrahedron, respectively or in the case the nucleotide base is equal to the previous nucleotide base, the nucleotide base is assigned to the same vertex as the vertex to which the previous nucleotide base has been assigned to ; and

c) determining for each vertex of the cube or the tetrahedron the number of nucleotides assigned to that vertex of the cube or the tetrahedron, respectively.

In a preferred embodiment, the first nucleotide is assigned to its corresponding vertex in a predetermined initial face of the cube or the tetrahedron.

In a further preferred embodiment, wherein the nucleotide sequence is represented in form of a cube, the predetermined initial face is the face comprising the nucleotide bases in G-A-T-C or G-A-U-C order in clockwise sense.

In an even further preferred embodiment, the sequence of nucleotides is further represented in form of a matrix. The method further comprises:

d) assigning each vertex of the cube or tetrahedron to an element of a matrix with at least as many elements as vertices the cube or the tetrahedron has, respectively and

e) assigning the total number of nucleotides bases assigned to each vertex of the cube or the tetrahedron to the value of the assigned element of the matrix.

In a preferred embodiment of the matrix representation embodiment, the matrix is a square matrix and preferably wherein the vertices of the cube or the tetrahedron are represented on the elements in the diagonals of the matrix. More preferably, when the nucleotide sequence is represented in form of a cube, the square matrix is preferably a 4x4 matrix.

In a preferred embodiment of the matrix representation embodiment, the sequence of nucleotides is represented in form of a cube and wherein the vertices of the cube are assigned to the matrix by the 3D projection of the cube on a 2D Euclidean plane defining an inner and external set of nucleotide bases each representing opposing faces of the cube, preferably wherein the projection axis is the one perpendicular to the predetermined initial face.

In a preferred embodiment of any of the embodiments of the first aspect of the invention, the received sequence of nucleotides further comprises information indicating the 5' and 3' ends of the sequence and wherein the nucleotides are sequentially assigned to a base in the cube or the tetrahedron in the 5'→3' sense.

A second aspect of the invention refers to a computer-implemented method of tagging a nucleotide sequence, by encoding the nucleotide sequence in form of a matrix according to any of the matrix representation method embodiments of the first aspect of the invention.

A third aspect of the invention refers to a computer-implemented method of determining a modification of a nucleotide sequence. The method comprises the steps of:

a) from at least a selected nucleotide sequence representing a template sequence and at least a selected sequence representing at least a nucleotide sequence for which the modification is to be determined, computing the matrix representation of the sequences according to any of the matrix representation method embodiments of the first aspect of the invention,

b) individually comparing the equivalent matrix elements of the at least one template sequence and the at least one sequence for which the modification is to be determined matrix representations, and

c) determining that a modification between the at least one template sequence and the at least one sequence for which the modification is to be determined has occurred when any of the equivalent matrix elements are not equal.

Alternatively, the method comprises the steps of:

a) from at least a selected nucleotide sequence representing a template sequence and at least a selected sequence representing at least a nucleotide sequence for which the modification is to be determined, computing the matrix representation of the first and second sequences according to any of the matrix representation method embodiments of the first aspect of the invention, wherein the matrix is a square matrix,

b) computing the determinant of the matrix representation of the sequences of step a),

c) comparing the determinants of the at least one template sequence and the at least one sequence for which the modification is to be determined matrix representation, and

d) determining that a modification between the at least one template sequence and the at least one sequence for which the modification is to be determined has occurred when the determinants of step c) are not equal.

In a preferred embodiment, the modification is one or more of a SNP, a nucleotide insertion, a nucleotide deletion or a nucleotide transposition.

In a further preferred embodiment of any of the embodiments of the third aspect of the invention, the method further comprises determining that the integrity of a biological sequence has been broken when a modification in the sequence has been determined.

A fourth aspect of the invention relates to computer-implemented method of defining one or more markers in a nucleotide sequence, the markers characterised by a symmetry invariance, the method comprising the steps:

a) receiving a sequence of nucleotides,

b) selecting a part of the sequence to be analysed whose base length is the square of an integer $n^2$,

c) splitting the sequence of base length $n^2$ into n fragments of length n

d) assembling the sequence into an n x n matrix

e) computing several transformations of the matrix using one or more symmetries, until all the feasible transformations for each of the selected symmetries are exhausted,

f) determining the elements of the matrices that are invariant across all transformations for each of the selected symmetries, and

g) assigning the invariant elements of the matrices as invariant marker.

h) repeating steps a) to g) using another part of the sequence, preferably by sliding the start of the sequence to be analysed by one or more nucleotides; until all the sequence is analysed.

In a preferred embodiment, the one or more symmetries includes a rotational and/or a mirror symmetry.

In another preferred embodiment, the n is an odd number n, preferably prime, more preferably greater or equal to 5.

A fifth aspect of the invention refers to a computer program product comprising instructions which, when the program is executed by a processor, cause the computer to carry out the steps of any of the methods according to the first, second, third and/or fourth aspect of the invention.

## Brief description of the drawings

To enable better understanding of the present disclosure, and to show how the same may be carried into effect, reference will now be made, by way of example only, to the accompanying schematic drawings, in which:

Fig. 1 shows the list representation, the triangle representation, the square representation and the matrix representation of a nucleotide sequence as well as the strand orientation of each representation according to one or more embodiments.

Fig. 2A shows the nonredundant tetranucleotides permutations of the GATC alphabet.

Fig. 2B shows the nonredundant trinucleotides permutations of the GATC alphabet.

Fig. 3 shows how the cube of nucleotides can be formed from six copies of the square representation, according to one or more embodiments.

Fig. 4 shows how the tetrahedron of nucleotides can be formed from four copies of the triangular representation, according to one or more embodiments.

Fig. 5A shows a cube of nucleotides bases according to one or more embodiments.

Fig. 5B shows the expansion of the square representation of the cube of Fig. 5A, according to one or more embodiments.

Fig. 5C shows the formation of a rhombicuboctahedron of nucleotides from the expansion of the square representation of Fig. 5B, according to one or more embodiments.

Fig. 6 shows the relationship of the cube and the octahedron through the rhombicuboctahedron, according to one or more embodiments.

Fig. 7A shows a perspective view of a genocube according to one or more embodiments.

Fig. 7B shows a planar view of a genocube according to one or more embodiments.

Fig. 7C shows another planar view of a genocube differentiating the internal and external sets of nucleotides according to one or more embodiments.

Fig. 8A shows an example of a sequence of nucleotides according to one or more embodiments.

Fig. 8B shows an example of a cube definition using nucleotide bases and an assignation of the sequence of nucleotides of Fig. 8A to the vertices of the cube according to one or more embodiments.

Fig. 8C shows an isometric 3D projection of the cube and the assignation of the sequence to the cube of Fig. 8B according to one or more embodiments.

Fig. 8D shows a matrix representation of the sequence of Fig 8A and its cube representation of Figs 8B and 8C.

Fig. 9 shows an example of a matrix encoding of a large nucleotide sequence according to one or more embodiments.

Fig. 10 shows an example the sensitivity of the matrix encoding to small changes according to one or more embodiments.

Fig. 11 shows a diagram of a process of determining invariant markers in a nucleotide sequence according to one or more embodiments.

Fig. 12. shows an example of a process of determining invariant markers in a nucleotide sequence according to one or more embodiments.

## Description of the invention

### Definitions

The term "symmetric group" refers to the group whose elements are all the bijections from the set to itself, and whose group operation is the composition of functions. In the particular case of this invention, the finite symmetric group $S_4$ defined over a finite set of 4 symbols consists of the permutations that can be performed on the 4 symbols. Since there are n! (n factorial) of such permutation operations, the order (number of elements) of the symmetric group S4 is 4! = 24.

The term "dihedral group" refers to the group formed by the different rotational and reflection symmetries of a regular polygon with n sides. There are n rotational symmetries and n reflection symmetries. The Dihedral group D4 is the group of symmetries of the square.

The term "alternating group" refers to the group of even permutations of a finite set. The alternating group on a set of n elements is called the alternating group of degree n, or the alternating group on n letters and denoted by An or Alt(n).

The term "genocube" refers to a cube where each nucleotide is represented twice, on diagonally opposite vertices of the cube.

**Description**

In this work, whether biological sequences encoded by genes & genomes contain symmetries, and consequently could be tackled using the mathematical power of abstract algebra and group theory has been explored. It was hypothesised that the apparent opposition between symmetrical and evolutionary transformations might in fact represent two distinct manifestations of the same phenomenon. It was firstly noticed that changes in nucleotide sequences (i.e., mutations) can be mimicked by mathematical rearrangements of an ordered set of nucleotides (i.e., permutations).

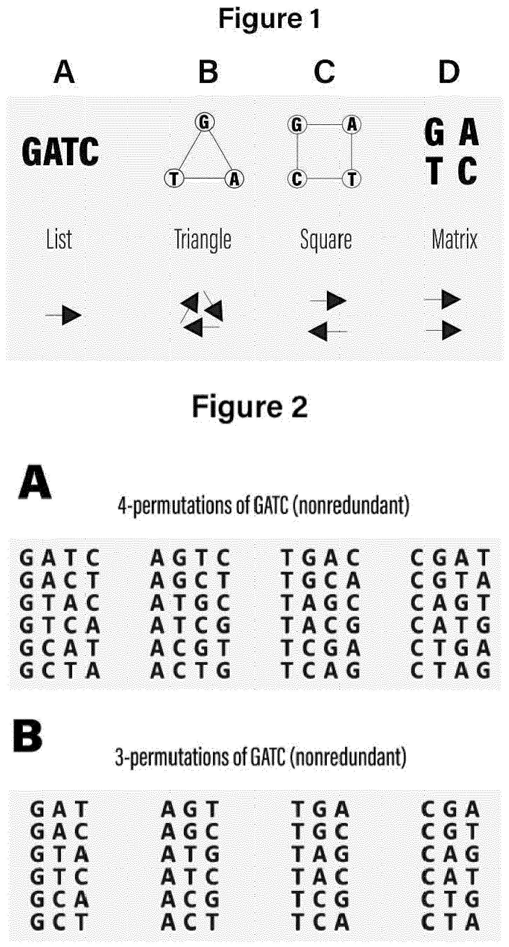

Although the work has been focused on the four letters used in DNA, the findings and methods herein described can be easily extendable to any 4-letter nucleotide alphabet such as the RNA one or other modified-nucleotide 4-letter alphabet. As reported in Fig. 1, the four letters of DNA can be represented in several distinct manners such as linear (A), circular (B & C), and tabular (D). These representations are equivalent information-wise since any polynucleotide can be represented in all three models, but they differ by their symmetrical properties. However, when inspected under the prism of symmetries, representations B, C and D presented some unique properties. The "List representation" does not contains symmetries a priori, (except for repeated, monotonous and/or palindromic sequences). The "Equilateral triangular representation" displays the dihedral symmetry of order 6. The "Square representation" and the "Matrix representation" both exhibit the dihedral symmetry of order 8, but display different strand orientation (antiparallel versus parallel).

Since biological sequences, including genetic sequences, are linear polymers of consecutive nucleotides, the permutations of the GATC was studied. As shown in Fig. 2, by making a systematic analysis of n-permutations (with n varying from 2 to 12) we found that, surprisingly, 3- and 4-permutations of GATC yielded permutation groups containing the same number of element (24). This observation can be explained by the existence of a universal mathematical structure, referred to as the Symmetric Group $S_n$, describing the number of possible permutations associated with a set of n-elements, as n!. In our case, the number of nonredundant 4-permutations of GATC is given by computing 4! = 4x3x2x1 = 24. Similarly, the number of nonredundant 3-permutations of GATC is given by the probabilistic formula 4x3x2 = 24. These two sets are in a bijective relationship with the symmetric group $S_4$ and are consequently isomorphic to this group. Since the group $S_4$ is isomorphic to the group of symmetries of the cube, this result establishes a one-to-one and onto correspondence between the groups of 3-, and 4-nucleotides permutations and the group symmetries of the cube. If we number the vertices of the cube from 1 to 4, and where opposite vertices are given the same number, the permutation corresponding to a symmetry can be read out from one of the faces.

Also of great interest is the fact that the $S_4$ group can be decomposed into three finite subgroups: (i) the Dihedral groups $D_3$, isomorphic to the group of symmetries of the triangle, (ii) the Dihedral groups $D_4$, isometric to the group of symmetries of the square, and the Alternating group ($A_4$), isomorphic to the group of symmetries of the regular tetrahedron. From a biological standpoint, the existence of these group isomorphisms means that any nucleotide sequence, composed of 3 and 4 permutations of the {G, A, T, C} alphabet, can be represented as a unique path through the vertices of a tetrahedron or a cube.

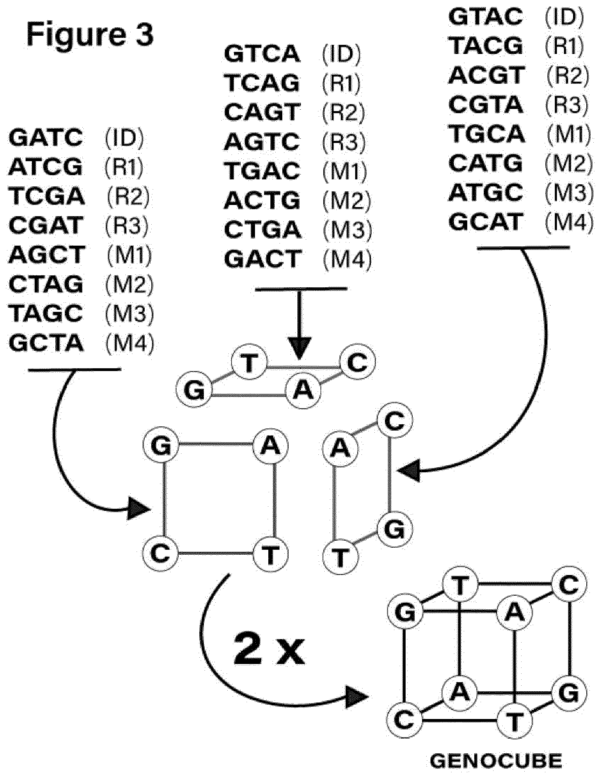

With respect to Fig. 3, it can be seen that when depicted using the "Square representation", the 24 nonredundant tetranucleotides permutations of the GATC alphabet fall into three subsets of 8 distinct permutations. Each subset contains the 8 dihedral symmetries corresponding to the symmetry group $D_4$. This group has 8 members, namely: one identity (ID), three rotational symmetries (R1 to R3), and three mirror symetries (M1 to M4). More interestingly, when represented twice, these 48 permutations give rise to a cube (referred to as the genocube) where each nucleotide is represented twice, on diagonally opposite vertices of the genocube. This representation is advantageous since any nucleotide sequence can be represented on such genocube. The resulting group (two copies of $S_4$) is isomorphic to the Octahedral symmetry group $(O_h)$ of order 48, resulting from the composition $(S_4 \circ \mathbb{Z}_2)$ the permutation ($S_4$) and the cyclic ( $\mathbb{Z}_2$ ) subgroups.

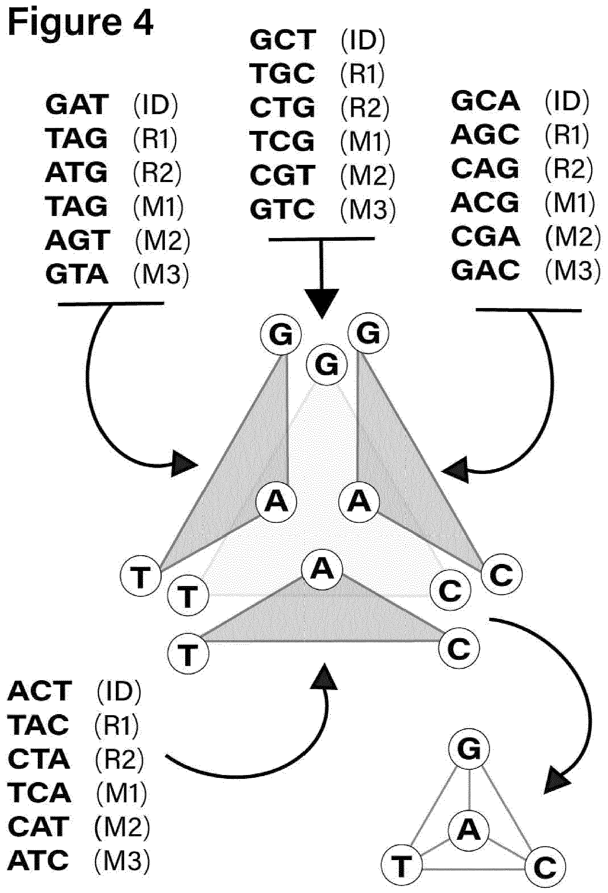

The same kind of reasoning was conducted with the set of 3-permutations. As depicted in Fig. 4, the 24 nonredundant 3-permutations of GATC can be represented by placing a single nucleotide on each of the vertices of an equilateral triangle i.e. the "Triangular representation". The resulting action classifies all 3-permutations into four subsets of 6 distinct permutations (6 x 4 =24), each permutation corresponding to a symmetry of the Dihedral group $D_3$ representing the group of symmetries of the equilateral triangle. This group has 6 members, namely: one identity (ID), two rotational symmetries (R1 to R2), and three reflection mirror symmetries (M1 to M3). This finding allowed us to assemble the resulting for equilateral triangles into a regular tetrahedron, which, as observed for the Genocube, harbours the property to represent any nucleotide sequence (DNA, RNA or sequences including modified nucleotides) in its vertices.

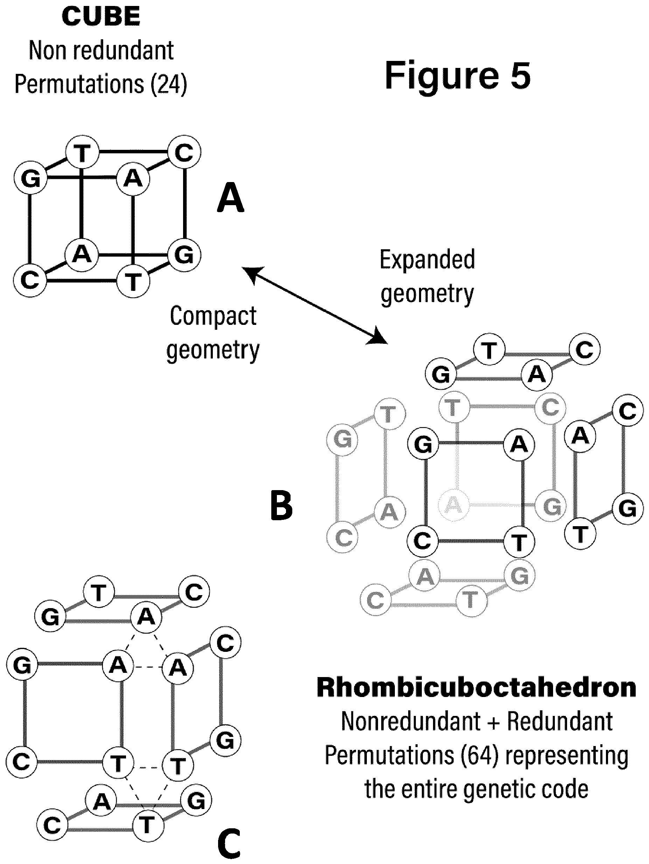

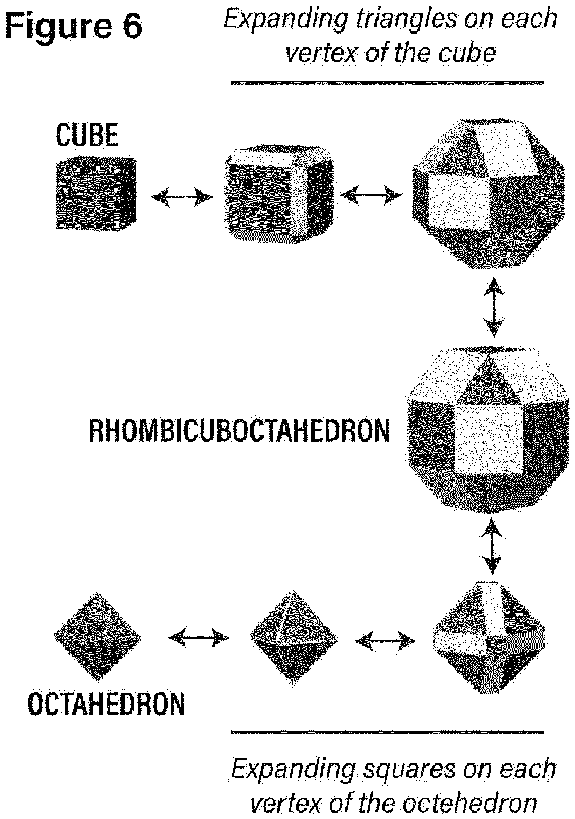

The 24 nonredundant permutations of GATC only cover a small subset of the genetic information that can be encoded in a redundant 3-letter code (24 *out* $4^3$ =64 possible words) or a 4-letter code (24 out $4^4$ =256 possible words). We note that this hurdle can be overcome since, when physically expanded by separating faces, the geometry of the cube (see Figs. 5A and 5B), create at each vertex, a multiplicity of the original nucleotide (see the example of triple As and Ts, presented in Fig. 5C). Therefore, 24 nonredundant 3-permutations and 4-permutations of the GATC alphabet can be extended to cover any redundant permutations (such as AAA, GAA, TTT, AAAA, GAAA, ATTT, etc.) by simply expanding the faces of the Genocube. This leads to the generation of three copies of the letter present in each vertex of each square. When connected, this transform the cube into a novel shape, intermediate between a cube and an octahedron, referred to as a rhombicuboctahedron. This rhombicuboctahedron can represent any of the 64 triplets constituting the Genetic code. This observation is supported by a mathematical property called Duality, that transforms topologically a cube into a regular octahedron by inflating triangles in each vertex of the cube. As shown in Fig. 6, the rhombicuboctahedron can then be transformed into a regular octahedron by vanishing the squares located on each vertex of the triangle. Conversely, an octahedron can be transformed into a rhombicuboctahedron by blowing a square in each of its vertices. To be noted that a regular octahedron has 24 rotational (or orientation-preserving) symmetries, and 48 symmetries altogether. These include transformations that combine a reflection and a rotation. A cube has the same set of symmetries, since it is the polyhedron that is dual to an octahedron.

Using this dual model, one can represent nonredundant permutations on the cube and/or the Tetrahedron and expand locally/temporarily the geometry to represent the rest of redundant permutations. Altogether, we hypothesize that this new representation will have profound implications for understanding the nature, structure, and evolution of the genetic code and genetic information in general.

Therefore, we herein describe a new way to compactly represent nucleotide sequences as a unique and virtual path along the vertices of a cube, or a tetrahedron.

A first aspect of the invention refers to, a computer-implemented method of representing a sequence of nucleotides. The method comprises the following steps:

a) Defining a cube or a tetrahedron using nucleotide bases, such that each vertex of the cube or the tetrahedron is assigned a nucleotide base. Each vertex is assigned a nucleotide base such that for each vertex, the assigned base is directly connected to every other base type through an edge. Therefore, when deciding which nucleotide base is assigned to a vertex, the bases of the vertices directed connected to such vertex through an edge must be considered, and a nucleotide base different from that of the directly connected vertices is assigned. As each vertex is directly connected to other three vertices through an edge, upon completion of the cube or tetrahedron definition, any vertex of interest will be directly connected to three vertices through an edge wherein the three vertices have different bases assigned between them and to the base assigned to the vertex of interest. It is noted that when the nucleotide sequence is represented in the form of a cube, each nucleotide base type is represented twice, on diagonally opposite vertices of the cube. Advantageously, this allows for any nucleotide sequence to be represented on such cube, as in all cases each subsequent nucleotide base can be either assigned to the same vertex as the one the previous nucleotide base has been assigned to, or to a vertex directly connected to the vertex the previous nucleotide base has been assigned to, as each vertex is directly connected to every other base type through an edge.

b) From a selected sequence of nucleotides, the sequence of nucleotides comprising nucleotides from one of the nucleotide subsets [A,C,G,T] or [A,C,G,U], assigning the first nucleotide base of the selected sequence of nucleotides to a vertex to which the base type of the nucleotide has been assigned and sequentially assigning each subsequent nucleotide of the selected sequence of nucleotides to a corresponding vertex in the cube or the tetrahedron, such that the assigned vertex is either directly connected to the vertex of the previous nucleotide base through an edge of the cube or the tetrahedron, respectively or in the case the nucleotide base is equal to the previous nucleotide base, the nucleotide base is assigned to the same vertex as the vertex to which the previous nucleotide base has been assigned to.

It is noted that any sequence of DNA, RNA or a set of four modified nucleotides may be therefore represented. It is also noted that in the tetrahedron there are only four vertices, so each vertex has a different nucleotide base assigned. Therefore, the first nucleotide is assigned to the only vertex to which the base type of the nucleotide has been assigned. However, in the cube case, there are eight vertices, so for each nucleotide base, there are two vertices to which the base type of the nucleotide has been assigned. Therefore, the first nucleotide base of the sequence of nucleotides may be assigned to any of the two vertices to which the base type of the nucleotide has been assigned.

c) Determining for each vertex of the cube or the tetrahedron the number of nucleotides assigned to that vertex of the cube or the tetrahedron, respectively. Therefore, a cube or tetrahedron representation with a of the sequence of nucleotides is obtained, with a value determined for each vertex of the cube or tetrahedron.

According to a preferred embodiment, wherein the nucleotide sequence is represented in the form of a cube or a tetrahedron, the first nucleotide is assigned to its corresponding vertex in a predetermined initial face of the cube or the tetrahedron, Advantageously, having a predetermined initial face in a cube solves the redundancy problem of the number of vertices to which the base type of the nucleotide has been assigned. By establishing a predetermined face, a consistent representation of any sequence in the form of a cube can be achieved. It is noted that this does not arise on the tetrahedron as the tetrahedron only has one vertex to which the first nucleotide base can be assigned, so there is no redundancy.

In another preferred embodiment, when the nucleotide sequence is represented in the form of a cube, the predetermined initial face is the face comprising the nucleotide bases in G-A-T-C or G-A-U-C order in clockwise sense. Advantageously, this provides a consistent selection of the predetermined initial face.

In order to further exploit the properties of such encoding, a matrix representation of the genocube can be designed. In a preferred embodiment of any of the previous embodiments, the sequence of nucleotides is further represented in form of a matrix. Advantageously, representing the nucleotide sequence in the form of a matrix enables the use of different matrix tools over the representation of the nucleotide sequence. Therefore, preferably, the method further comprises the steps of:

d) assigning each vertex of the cube or tetrahedron to an element of a matrix with at least as many elements as vertices the cube or the tetrahedron has, respectively. In the case when the nucleotide sequence is represented in the form of a tetrahedron, each vertex of the tetrahedron is assigned to an element of a matrix with at least 4 elements. In the case when the nucleotide sequence is represented in the form of a cube, each vertex of the tetrahedron is assigned to an element of a matrix with at least 8 elements.

e) assigning the total number of nucleotides bases assigned to each vertex of the cube or the tetrahedron to the value of the assigned element of the matrix. Therefore, a matrix comprising at least the number of nucleotides of each vertex of the cube or the tetrahedron is obtained in representation of the sequence of nucleotides.

From herein after, the matrix representing the vertices of a genocube may be referred to as TISA (Topologically Invariant Sequence Array).It is noted that the expression topologically invariant in the context of this invention refers to that the sequence identity is left unchanged through various transformation and/or representations, i.e. that the matrix is just another representation of a sequence of amino acids.

It is noted that several designs may be foreseen when assigning the vertices of the cube or the tetrahedron to a matrix, as multiple choices can be made when deciding how the vertices shall be assigned to each element in the matrix representing a vertex. For example, in the case when the nucleotide sequence is represented in the form of a tetrahedron, a matrix comprising at least 4 elements may be shaped at least as a (files by columns) 4x1, 2x2 or 1x4 matrix. In the case when the nucleotide sequence is represented in the form of a cube, the number of sizes of the matrix can expand. Moreover, multiple matrix designs can fulfil the requirement of comprising at least as many elements as vertices the cube or the tetrahedron has. Therefore, in the case when the nucleotide sequence is represented in the form of a cube, the sequence may be represented using a 4x4 matrix, wherein half of the elements of the matrix (8) have a vertex of the cube assigned, while the other half (8) doesn't have a vertex of the cube assigned.

In a more preferred embodiment, the matrix is a square matrix. Advantageously, a square matrix has further properties, such as that the determinant may be computed.

In a further preferred embodiment, when the nucleotide sequence is represented in the form of a cube, the sequence is represented by a 4x4 matrix. In an even further preferred embodiment, the vertices of the of the cube or the tetrahedron are represented on the elements in the diagonals of the matrix. Advantageously, this ensures that whenever the sequence is winded through all the vertices of the cube at least once, the matrix determinant will not be 0. In another even further preffered embodiment, when the nucleotide sequence is represented in the form of a cube, the cube vertices are assigned to a square 4 by 4 matrix wherein the vertices values are assigned to its diagonal elements.

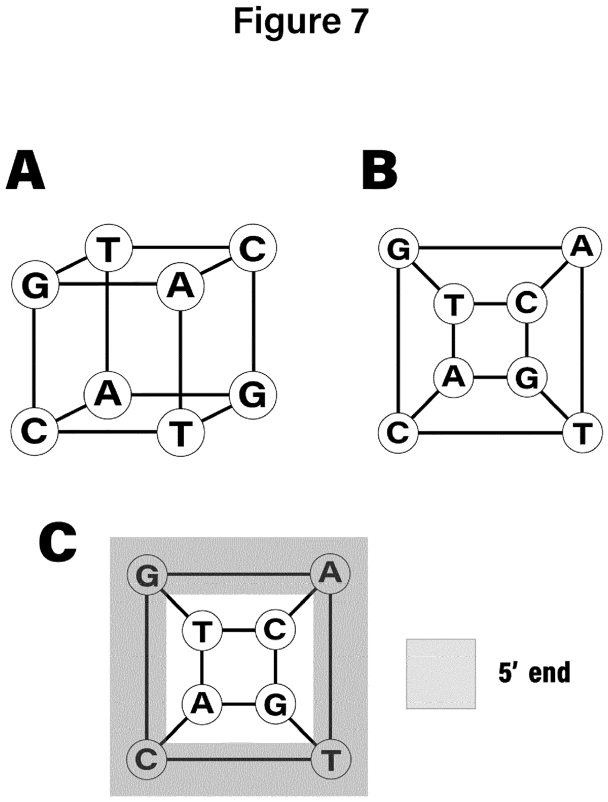

As shown in Fig. 7A, a cube can be represented using isometric 3D projection or as shown in Fig. 7B projected on the 2D Euclidean plane. Interestingly, the 2D projection transforms the cube into a plannar graph that defines an exterior and an interior set of nucleotide bases as shown in Fig. 7C, wherein the external set of nucleotides is highlighted. This property is interesting since it mimmicks the intrinsic polarity of polynucleotide sequences.

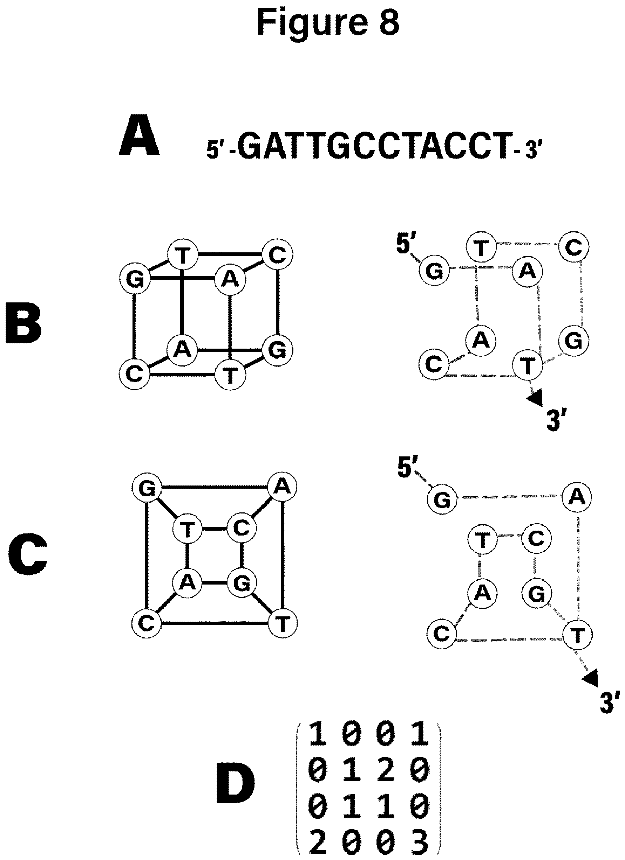

In a more preffered embodiment of any of the previous preffered embodiments wherein the sequence of nucleotides is represented in form of a cube, the vertices of the cube are assigned to the matrix by the 3D projection of the cube on a 2D Euclidean plane defining an inner and external set of nucleotide bases each representing opposing faces of the cube. Advantageously, this allows for a consistent representation of the cube in a square 4x4 matrix wherein the vertices values are assigned to its diagonal elements, as shown for example in Fig. 8D. More preferably, the projection axis is the one perpendicular to the predetermined initial face.

In each sequences of nucleotides, a 5' and a 3' end may be defined. In another preffered embodiement of any of the previous embodiments, the received sequence of nucleotides further comprises information indicating the 5' and 3' ends of the sequence and wherein the nucleotides are sequentially assigned to a base in the cube or the tetrahedron in the 5'→3' sense. Advantageously, this further prevents that two different representations of the same sequence may be obtained when no consideration of the starting end is. It is noted that alternatively, the nucleotides may be sequentially assigned to a base in the cube or the tetrahedron in the 3'→5' sense.

Given a particular nucleotide sequence, the nucleotide sequence may be tagged so that it can be easily identified among other sequences. The representation method through a matrix herein described, can serve as a way of tagging sequences. Therefore, a second aspect of the invention refers to a computer-implemented method of tagging sequences using a matrix representation by encoding the nucleotide sequence in form of a matrix, according to any of the methods described in the first aspect of the invention.

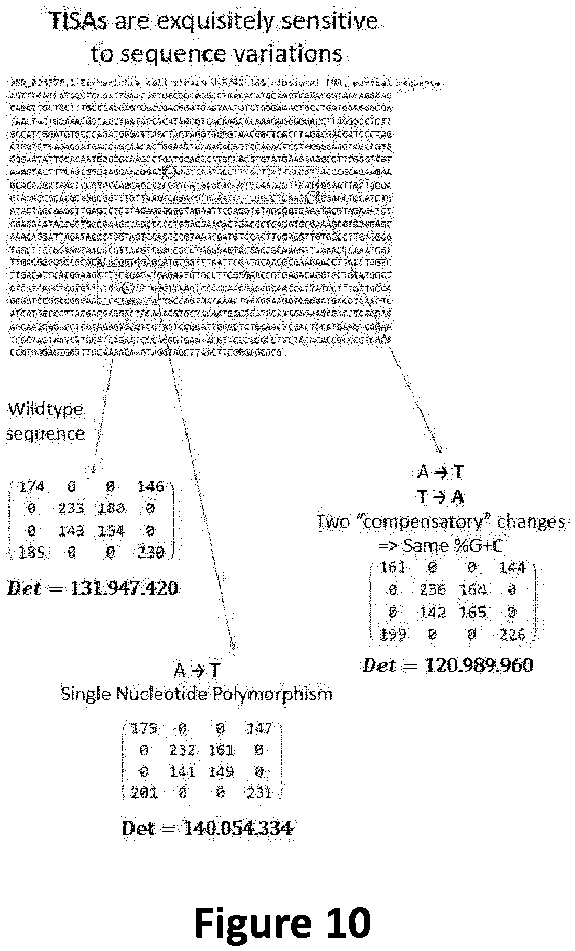

A third aspect of the invention refers to a computer-implemented method of determining a modification of a nucleotide sequence. Deep sequencing, the human genome polymorphisms, or the study of pangenomes are activities that require to handle large amount of nucleotide sequences. During the process of handling, exchanging, or storing this information, errors can appear and threaten the quality of the conclusions extracted from these data. As represented in Fig. 10 and in Example 2, TISA matrices are exquisitely sensitive to sequence variation. Indeed, any change, even minor (SNP) will change the winding path of the sequence considered and will consequently change the structure of the TISA matrix and the result of its determinant. Similarly, TISAs can be used to represent and score genetic mutations between related or unrelated sequences.

The computer-implemented method of determining a modification of a nucleotide sequence comprises the following steps:

a) from at least a selected nucleotide sequence representing a template sequence and at least a selected sequence representing at least a nucleotide sequence for which the modification is to be determined.; computing the matrix representation of the sequences according to any of the methods of the first aspect of the invention wherein the sequence of nucleotides is represented in form of a matrix. Preferably he template sequence is the expected sequence of the sequence for which the modification is to be determined sequence.

b) Individually comparing the equivalent matrix elements of the at least one template sequence and the at least one sequence for which the modification is to be determined matrix representations. The equivalent matrix elements of each matrix are the elements positioned in the same position (i.e. file and row) of the represented matrix.

c) Determining that a modification between the at least one template sequence and the at least one sequence for which the modification is to be determined has occurred when any of the equivalent matrix elements are not equal.

It is noted that although perfectly palindromic sequences as well as sequences having the same composition 5->3' and 3'-5' will have the same TISA signature, whenever any of the equivalent matrix elements are not equal there is for sure a modification between the first sequence and the second sequence.

Alternatively, the computer-implemented method of determining a modification of a nucleotide sequence comprises the following steps:

a) From at least a selected nucleotide sequence representing a template sequence and at least a selected sequence representing at least a nucleotide sequence for which the modification is to be determined. T

computing the matrix representation of the sequences according to any of the methods of the first aspect of the invention wherein the sequence of nucleotides is represented in form of a square matrix, Preferably he template sequence is the expected sequence of the sequence for which the modification is to be determined sequence.

b) Computing the determinant of the matrix representation of the sequences.

c) Comparing the determinants of at least one template sequence and the at least one sequence for which the modification is to be determined matrix representations.

d) Determining that a modification between the at least one template sequence and the at least one sequence for which the modification is to be determined has occurred when the determinants of step c) are not equal.

Similarly, it is noted that although perfectly palindromic sequences as well as sequences having the same composition 5->3' and 3'-5' will have the same TISA signature, and that there is a minimum possibility of two completely different matrices having the same determinant, whenever the determinants are not equal there is for sure a modification between the first sequence and the second sequence.

Advantageously, using a determinant instead of the equivalent elements of the TISA matrices, further simplifies the comparison process and the relevant information can be further compressed, by only assigning a determinant to each sequence.

In a preffered embodiment of any of the embodiemnts of the third aspect of the invention, the modification is one or more of a SNP, a nucleotide insertion, a nucleotide deletion or a nucleotide transposition. As shown in Example 2, a nucleotide insertion, a nucleotide deletion or a nucleotide transposition during the storage/recovery or exchange of the sequence of nucleotides through a network, will generate a different TISA matrix.

Therefore, in another preffered embodiement of any of the embodiments of the third aspect of the invention, the method further comprises the step of determining that the integrity of a biological sequence has been broken when a modification in the sequence has been determined. As also shown in Example 2, TISA matrix representation is a good checksum system, as any change due to information loss, noise, errors, will generate a different TISA matrix, so when a biological sequence has been broken, it can be determined by comparing the current TISA matrix with the previous one, or the current determinant with the previous one.

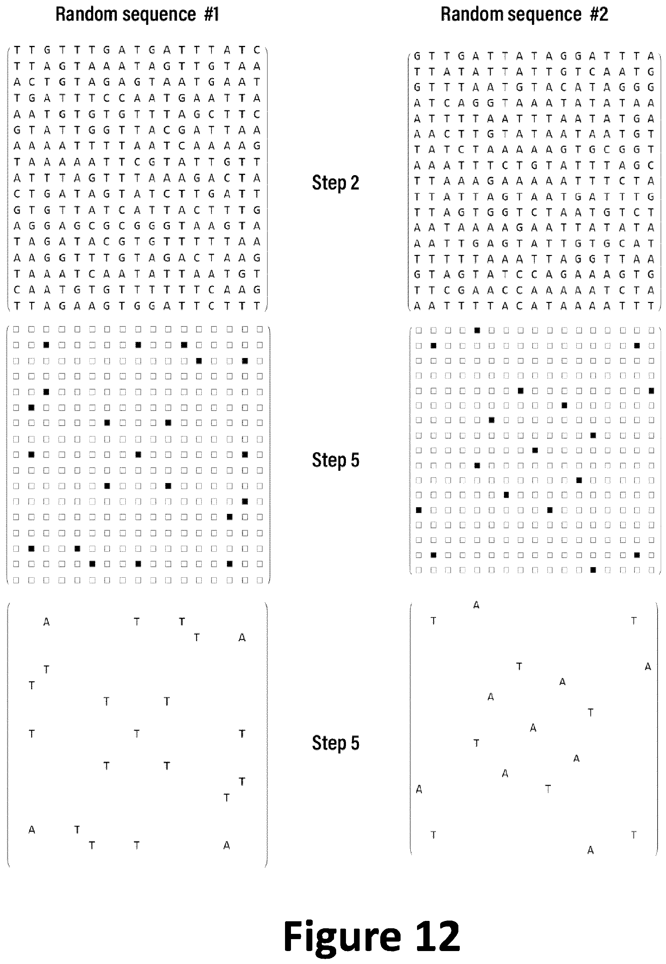

After discovering symmetric laws embedded inside the DNA alphabet, the presence of nucleotides or sequences invariant to transformations by symmetries was analysed. For that purpose, the RIP, MIP RMIP methodologies were devised, standing respectively for Rotational (R), Mirror (M), or Rotational + Mirror (RM) Invariant Patterns (IP). The principle of all these methodologies is exemplified with RIP in Fig. 12 but is similar for MIPs and RMIPs (the only change stands in the nature of the symmetry to be applied).

A fourth aspect of the invention refers to a computer-implemented method of defining one or more markers in a nucleotide sequence, the markers characterised by a symmetry invariance. . The method comprises the steps:

a) Receiving a sequence of nucleotides, the sequence of nucleotides comprising nucleotides from one of the nucleotide subsets [A,C,G,T] or [A,C,G, U]. It is noted that any sequence of DNA, RNA or a set of four modified nucleotides may be therefore represented.

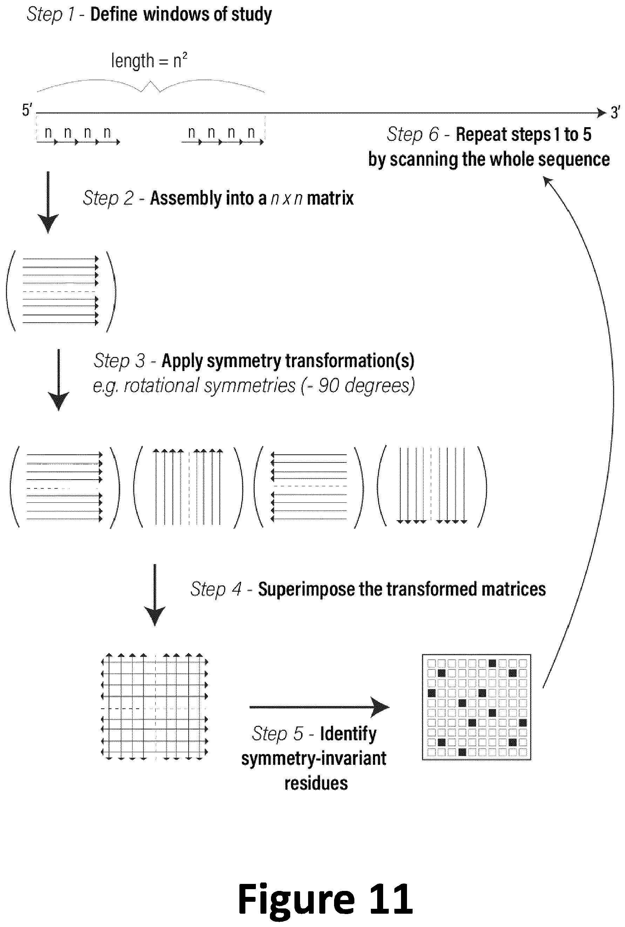

b) Selecting a part of the sequence as a window to be analysed whose base length is the square of an integer $n^2$. It is noted that the sequence of nucleotides base length must be at least $n^2$. This can be seen in Fig. 11 step 1.

c) Splitting the sequence of base length $n^2$ into n fragments of length n. It is noted that the sequence is split into n fragments, so that each fragment first nucleotide is the consecutive nucleotide of the last nucleotide of the previous fragment.

d) Assembling the sequence into an n x n matrix. The matrix is preferably assembled into a matrix by placing each sequence fragment as a row in the matrix, and placing consecutive fragments in consecutive row. However, it is noted that other obvious assembling strategies such as placing the fragments as columns or using other distribution of the fragments, may be used. This can be seen in Fig. 11 step 2.

e) Computing several transformations of the matrix using one or more of the symmetries until all the feasible transformations for each of the selected symmetries are exhausted. This can be seen in Fig. 11 step 3.

f) Determining the elements of the matrices that are invariant across all transformations for each of the selected symmetries. To do this, the equivalent matrix elements in each of the matrix symmetries are compared, and the elements that are invariant across all transformations are determined as such. This can be seen in Fig. 11 steps 4 and 5.

g) Assigning the invariant elements of the matrices as invariant marker.

h) Repeating steps a) to g) using another part of the sequence, preferably by sliding the start of the sequence to be analysed by one or more nucleotides; until all the sequence is analysed. This can be seen in Fig. 11 step 6.

The RIP methodology, as well as its derived variations (MIPs & RMIPs), are advantageous since they can identify and distinguish markers and genetic landmarks in a single nucleotide sequence, in absence of any polymorphism. Thus, the RIP, MIPs and RMIPs methodologies are suitable tools to study the structure, function, and evolution of genes and genomes.

In another preffered embodiement, the one or more symmetries are any of a rotational and/or a mirror symmetry. As shown in and Example 3, a square matrix has several rotational and mirror simmetries. It is noted that for a matrix has three rotational symmetries (see Fig. 11) and four mirror symmetries, (across each diagonal and through the mid row and mid column). Any combinations of rotational and/or mirror symmetry may be applied.

Interestingly, the number of invariant markers is a multiple of the number of transformation applied and optioanlly one more invariant corresponding to the rotation center. Note that for the RIPs in Fig. 12 the number of invariant markers is a multiple of 4 since 3 rotations + identity (rotation centre).

In a preffered embodiment of any of the embodiments of the fourth aspect of the invention, the the n is preferably an odd number n, more preferably prime, more preferably greater or equal to 5. It is noted that when n is an odd nuber, the matrix will be centred around a nucleotide, thus a invariant element will be always found in the central element of the matrix (n/2, n/2). This element should not be considered as a marker.

Since RIPs, MIPs and RMIPs can deal with any biological sequence (nucleotides, proteins, epigenetic markers, sugars, etc.) they are a choice to study genotypes, phenotypes, and biological traits. Indeed, as of previously described markers such as SNPs, RFLPs, Mass Spectrometry signatures, etc., they can be used to establish associations between the presence or absence of a given invariant marker, and a given biological property associated with health prevention, disease detection, breeding, population studies, etc.

The presence or absence of invariant markers and invariant marker patterns may be linked to a physiological condition. Moreover, there might be an correlation between the presence or absence of one or more potential biological markers on one or more n x n matrices with the expression of a given genotype, phenotype or biological trait. Therefore, an invariant marker may be used to indicate a physiological condition and/or the expression of a given genotype, phenotype or biological trait if a strong correlation is found.

The computer-implemented methods of any of the previous embodiments of the first, second, third and/or fourth aspects of the invention may be performed on any suitable computing system comprising one or more processing units, such as a microprocessor, GPU, CPU, multicore processor or similar, a server or a distributed computing system such as the cloud.

A fifth aspect of the invention refers to a computer program product comprising instructions which, when the program is executed by a processor, cause the computer to carry out the steps of any of the methods according to the first, second, third and/or fourth aspect of the invention. The computer program product may be implemented on hardware and/or software.

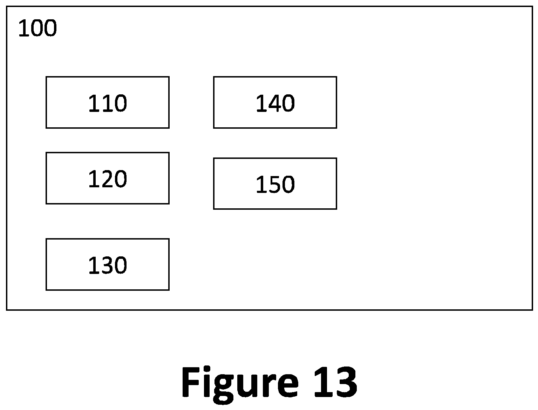

Figure 13 shows a computer program product 100 to implement the methods disclosed in the present document, that may be implemented in software, hardware or a combination thereof. The module may be saved in a memory of the system, or may be saved remotely, for example, in a remote server or distributed computing system communicatively connected to the system. The memory may comprise one or more volatile or non-volatile memory devices, such as DRAM, SRAM, flash memory, read-only memory, ferroelectric RAM, hard disk drives, floppy disks, magnetic tape, optical discs, or similar.

In some embodiments, the module may comprise a representation module 110 configured to represent a sequence of nucleotides as a cube or a tetrahedron and optionally as a matrix, according to any of the embodiments of the first aspect of the invention.

In some embodiments, the module may comprise a tagging module 120 to assign a matrix to sequence of nucleotides, by receiving from the representation module 110 the matrix representing the sequence nucleotide and assigning it to the sequence nucleotide according to the second aspect of the invention.

In some embodiments, the module may comprise a modification determination module 130 configured to determine a modification of a sequence of nucleotides by comparing it to a template sequence, according to any of the embodiments of the third aspect of the invention.

In some embodiments, the module may comprise a transformation module 140 configured to obtain matrices resulting from applying symmetry transformations to a square matrix.

In some embodiments, the module may comprise an invariance determining module 150 configured to superpose an original matrix and the transformed matrices from the transformation module and computing the elements of the matrices (same row and column) that are equal across all the matrices.

**Examples**

**Example 1: Example of a sequence matrix encoding**

As a first example, the encoding of a nucleotide sequence into a matrix has been performed in a step by step process, as shown in Fig.s 8A-8D.

The initial nucleotide sequence in the 5'→ 3' sense is: GATTGCCTACCT as shown in Fig. 8A. Then, in fig. 8B a cube is define, wherein each base is connected to every other base type through an edge. Then, the frontal face of the cube is defined as the predetermined initial face, and the sequence is winded along the vertices, such that the assigned base is connected to the base of the previous nucleotide through an edge of the cube. Then in fig. 8C, the 3D projection of the cube on a 2D Euclidean plane defining an inner and external squares is shown, as well as how the sequence winding would look form that perspective. Finally, in Fig. 8D, the number of nucleotides assigned to each base of the cube is assigned to such vertex of the cube, respectively, and the nucleotide sequence is encoded in a square matrix, wherein the vertices are represented on the elements in the diagonals of the matrix. The square matrix is a Topologically Invariant Sequence Array (TISA).

It is noted that the TISA matrix preserves the topology of the genocube. When the sequence to be encoded contains stretches of repeated nucleotides (e.g. TT or CC in example A) redundancy is captured by computing 2 units at the corresponding vertex. The sum of all positions in the TISA matrix is identical to the length of the encoded nucleotide sequence (here 12 nucleotides).

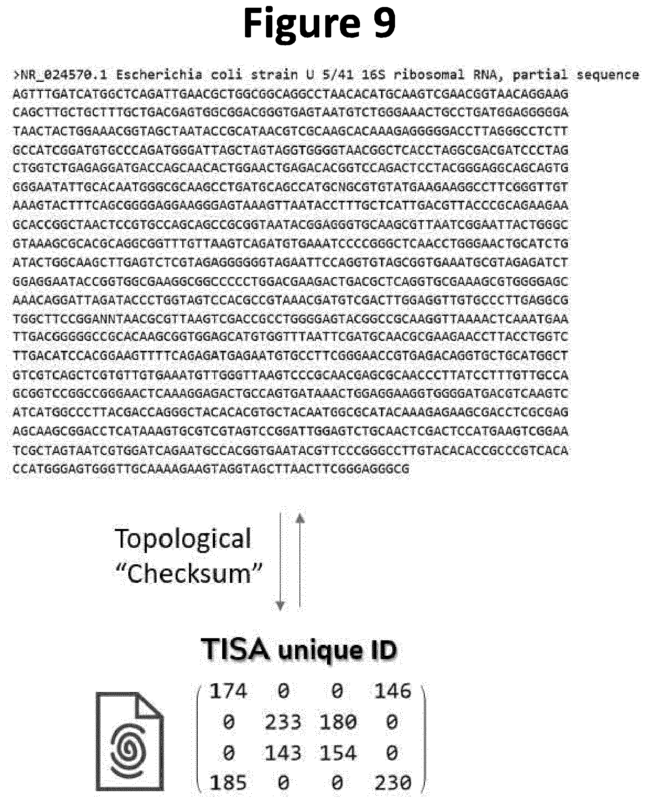

Moreover, Fig. 9 shows the TISA matrix of a more complex nucleotide sequence, a partial sequence of the Escherichia coli strain U 5/41 16S ribosomal RNA (Accession number NR_024570.1). The final TISA matrix is a 4 by 4 square matrix with the vertices of the cube represented on the elements in the diagonals of the matrix. TISA winding of a real DNA sequence around the Genocube (cube) exemplifies the level of compression and compactness obtained by this method.

This shows that a nucleotide sequence can be encoded through a matrix while preserving the topoligical information of the winded nucleotide segment in form of a cube. It is noted that the same applies to the winding over a tetrahedron, where a simple matrix would be obtained.

**Example 2: Example of the sensitivity of matrix encoding to changes.**

A second example is directed to show the high sensitivity of the proposed matrix encoding to small sequence changes according to one or more embodiments.

As shown in Fig. 10, the elements of the TISA matrix from the sequence introduced in Fig. 9 (a partial sequence of the Escherichia coli strain U 5/41 16S ribosomal RNA (Accession number NR_024570.1)), suffer significant changes when modifications, even if small are applied.

First, two compensatory changes were produced, by substituting the position of two base, so that the same percentage of each base is present in the sequence. It can be noticed that all elements of the matrix representing a vertex change.

Secondly, an even smaller change was tested. A single nucleotide polymorphism was tested (i.e changing one nucleotide by another with a different base). Equally, all elements of the matrix representing a vertex change.

Although the changes in the TISA matrix are noticeable, these can be further noticed when the determinant of each of the TISA matrices is computed. For the compensatory changes, the determinant decreased in more than 11 million, while for the single nucleotide polymorphism, it increased in more than 8 million.

Therefore it can be seen that the unique ID (either the TISA matrix or its determinant) obtained can be used to check the integrity of a nucleotide sequence during storage/recovery or exchange through a network. Any change due to information loss, noise, errors, will generate a different TISA matrix. Since the TISA matrix preserve the topoligical information of the winded nucleotide segment, it can be handle as a compact representation of the encoded sequence/gene/genome. Therefore, TISA encoding can serve as a way of tagging sequences. Moreover, this further shows that TISA encoding can be used to study the effect of mutations on nucleotide sequence, by representing and scoring genetic mutations between related or unrelated sequences.

**Example 3: Example of a determination of a invariant marker in a sequence of nucleotides by rotation symmetry (RIP)**

Finally, a fourth example showing different Rotational Invariant Patterns (RIPs) in two randomely generated nucleotide sequences is shown.

In the given sequences, a window length n of 17 has been preselected, according to one or more embodiments. The 17 by 17 matrices are therefore initially given in Fig. 13 (step 2).After applying several rotation symmetry, the symmetry invariant elements are identified, from which the positional information, this is, the position in the matrix of the symmetry invariant elements and the qualitative value, this is, the nature of the element (base type) can be obtained.

It is noted, that as the n is odd, the center of the matrix is always invariant (center of rotation). This element needs to be discarded since it is not informative per se. If a smaller n had been chosen, alternative matrices could have been built and different symmetry invariant elements located. Simmilarly,in a longer sequence, further RIPs could be computed by shifting the initial nucleotide.It is noted that this example refers to RIPs but the same considerations may apply to other variations sucgh as Mirror Invariant Patterns (MIPs) and Rotational Mirror Invariant Patterns (RMIPs).

RIP, MIPs and RMIPs methodologies may be suitable tools to study the structure, function, and evolution of genes and genomes.

**Claims**

1. A computer-implemented method of representing a sequence of nucleotides, comprising the steps of:

   a) defining a cube or a tetrahedron using nucleotide bases, wherein each vertex of the cube or the tetrahedron is assigned a nucleotide base, wherein each of the vertices is assigned a nucleotide base such that for each vertex, the assigned base is directly connected to every other base type through an edge,
   b) from a selected sequence of nucleotides, the sequence of nucleotides comprising nucleotides from one of the nucleotide subsets [A,C, G,T] or [A,C,G,U], assigning the first nucleotide base of the selected sequence of nucleotides, to a vertex to which the base type of the nucleotide has been assigned and sequentially assigning each subsequent nucleotide of the selected sequence of nucleotides to a corresponding vertex in the cube or the tetrahedron, such that the assigned vertex is either directly connected to the vertex of the previous nucleotide base through an edge of the cube or the tetrahedron, respectively; or in the case the nucleotide base is equal to the previous nucleotide base, the nucleotide base is assigned to the same vertex as the vertex to which the previous nucleotide base has been assigned to, and
   c) determining for each vertex of the cube or the tetrahedron the number of nucleotides assigned to that vertex of the cube or the tetrahedron, respectively.

2. The method according to claim 1, the first nucleotide is assigned to its corresponding vertex in a predetermined initial face of the cube or the tetrahedron.

3. The method according to claim 2, wherein the nucleotide sequence is represented in form of a cube and wherein the predetermined initial face is the face comprising the nucleotide bases in G-A-T-C or G-A-U-C order in clockwise sense.

4. The method according to the any of the claims 1 to 3, wherein the sequence of nucleotides is further represented in form of a matrix, further comprising:

d) assigning each vertex of the cube or tetrahedron to an element of a matrix with at least as many elements as vertices the cube or the tetrahedron has, respectively and

e) assigning the total number of nucleotides bases assigned to each vertex of the cube or the tetrahedron to the value of the assigned element of the matrix .

**5.** The method according to claim 4, wherein the matrix is a square matrix and preferably wherein the vertices of the cube or the tetrahedron are represented on the elements in the diagonals of the matrix.

**6.** The method according to claim 5, wherein the nucleotide sequence is represented in form of a cube, wherein the square matrix is preferably a 4x4 matrix.

**7.** The method according to claim 6, wherein the sequence of nucleotides is represented in form of a cube and wherein the vertices of the cube are assigned to the matrix by the 3D projection of the cube on a 2D Euclidean plane defining an inner and external set of nucleotide bases each representing opposing faces of the cube, preferably wherein the projection axis is the one perpendicular to the predetermined initial face.

**8.** The method according to any of the previous claims, wherein the received sequence of nucleotides further comprises information indicating the 5' and 3' ends of the sequence and wherein the nucleotides are sequentially assigned to a base in the cube or the tetrahedron in the 5'→3' sense.

**9.** A computer-implemented method of tagging a nucleotide sequence, by encoding the nucleotide sequence in form of a matrix, according to any of the methods of claims 4 to 8.

**10.** A computer-implemented method of determining a modification of a nucleotide sequence, the method comprising the steps of:

a) from at least a selected nucleotide sequence representing a template sequence and at least a selected sequence representing at least a nucleotide sequence for which the modification is to be determined, computing the matrix representation of the sequences according to any of the methods of claims 4 to 8,
b) individually comparing the equivalent matrix elements of the at least one template sequence and the at least one sequence for which the modification is to be determined matrix representations, and
c) determining that a modification between the at least one template sequence and the at least one sequence for which the modification is to be determined has occurred when any of the equivalent matrix elements are not equal.

**11.** A computer-implemented method of determining a modification of a nucleotide sequence, the method comprising the steps of:
from at least a selected nucleotide sequence representing a template sequence and at least a selected sequence representing at least a nucleotide sequence for which the modification is to be determined, computing the matrix representation of the sequences according to any of the methods of claims 5 to 8,

b) computing the determinant of the matrix representation of the sequences of step a),
c) comparing the determinants of the at least one template sequence and the at least one sequence for which the modification is to be determined matrix representations, and
d) determining that a modification between the at least one template sequence and the at least one sequence for which the modification is to be determined has occurred when the determinants of step c) are not equal.

**12.** The method according to claim 10 or 11, wherein the modification is one or more of a SNP, a nucleotide insertion, a nucleotide deletion or a nucleotide transposition.

**13.** The method according to any of the claims 10 to 12, further comprising determining that the integrity of a biological sequence has been broken when a modification in the sequence has been determined.

# Figure 1

| A | B | C | D |
|---|---|---|---|
| GATC | | | |
| List | Triangle | Square | Matrix |

# Figure 2

**A**

### 4-permutations of GATC (nonredundant)

```
G A T C     A G T C     T G A C     C G A T
G A C T     A G C T     T G C A     C G T A
G T A C     A T G C     T A G C     C A G T
G T C A     A T C G     T A C G     C A T G
G C A T     A C G T     T C G A     C T G A
G C T A     A C T G     T C A G     C T A G
```

**B**

### 3-permutations of GATC (nonredundant)

```
G A T     A G T     T G A     C G A
G A C     A G C     T G C     C G T
G T A     A T G     T A G     C A G
G T C     A T C     T A C     C A T
G C A     A C G     T C G     C T G
G C T     A C T     T C A     C T A
```

# Figure 3

GATC (ID)
ATCG (R1)
TCGA (R2)
CGAT (R3)
AGCT (M1)
CTAG (M2)
TAGC (M3)
GCTA (M4)

GTCA (ID)
TCAG (R1)
CAGT (R2)
AGTC (R3)
TGAC (M1)
ACTG (M2)
CTGA (M3)
GACT (M4)

GTAC (ID)
TACG (R1)
ACGT (R2)
CGTA (R3)
TGCA (M1)
CATG (M2)
ATGC (M3)
GCAT (M4)

2 x

GENOCUBE

# Figure 4

**GCT** (ID)
**TGC** (R1)

**GAT** (ID)
**TAG** (R1)
**ATG** (R2)
**TAG** (M1)
**AGT** (M2)
**GTA** (M3)

**CTG** (R2)
**TCG** (M1)
**CGT** (M2)
**GTC** (M3)

**GCA** (ID)
**AGC** (R1)
**CAG** (R2)
**ACG** (M1)
**CGA** (M2)
**GAC** (M3)

**ACT** (ID)
**TAC** (R1)
**CTA** (R2)
**TCA** (M1)
**CAT** (M2)
**ATC** (M3)

**CUBE**
Non redundant
Permutations (24)

**A**

**Figure 5**

Compact
geometry

Expanded
geometry

**B**

**C**

**Rhombicuboctahedron**
Nonredundant + Redundant
Permutations (64) representing
the entire genetic code

# Figure 6

*Expanding triangles on each vertex of the cube*

CUBE

RHOMBICUBOCTAHEDRON

OCTAHEDRON

*Expanding squares on each vertex of the octehedron*

# Figure 7

5' end

# Figure 8

**A** 5'-GATTGCCTACCT-3'

**B**

**C**

**D** $\begin{pmatrix} 1 & 0 & 0 & 1 \\ 0 & 1 & 2 & 0 \\ 0 & 1 & 1 & 0 \\ 2 & 0 & 0 & 3 \end{pmatrix}$

# Figure 9

```
>NR_024570.1 Escherichia coli strain U 5/41 16S ribosomal RNA, partial sequence
AGTTTGATCATGGCTCAGATTGAACGCTGGCGGCAGGCCTAACACATGCAAGTCGAACGGTAACAGGAAG
CAGCTTGCTGCTTTGCTGACGAGTGGCGGACGGGTGAGTAATGTCTGGGAAACTGCCTGATGGAGGGGGA
TAACTACTGGAAACGGTAGCTAATACCGCATAACGTCGCAAGCACAAAGAGGGGGACCTTAGGGCCTCTT
GCCATCGGATGTGCCCAGATGGGATTAGCTAGTAGGTGGGGTAACGGCTCACCTAGGCGACGATCCCTAG
CTGGTCTGAGAGGATGACCAGCAACACTGGAACTGAGACACGGTCCAGACTCCTACGGGAGGCAGCAGTG
GGGAATATTGCACAATGGGCGCAAGCCTGATGCAGCCATGCNGCGTGTATGAAGAAGGCCTTCGGGTTGT
AAAGTACTTTCAGCGGGGAGGAAGGGAGTAAAGTTAATACCTTTGCTCATTGACGTTACCCGCAGAAGAA
GCACCGGCTAACTCCGTGCCAGCAGCCGCGGTAATACGGAGGGTGCAAGCGTTAATCGGAATTACTGGGC
GTAAAGCGCACGCAGGCGGTTTGTTAAGTCAGATGTGAAATCCCCGGGCTCAACCTGGGAACTGCATCTG
ATACTGGCAAGCTTGAGTCTCGTAGAGGGGGGTAGAATTCCAGGTGTAGCGGTGAAATGCGTAGAGATCT
GGAGGAATACCGGTGGCGAAGGCGGCCCCCTGGACGAAGACTGACGCTCAGGTGCGAAAGCGTGGGGAGC
AAACAGGATTAGATACCCTGGTAGTCCACGCCGTAAACGATGTCGACTTGGAGGTTGTGCCCTTGAGGCG
TGGCTTCCGGANNTAACGCGTTAAGTCGACCGCCTGGGGAGTACGGCCGCAAGGTTAAAACTCAAATGAA
TTGACGGGGGCCGCACAAGCGGTGGAGCATGTGGTTTAATTCGATGCAACGCGAAGAACCTTACCTGGTC
TTGACATCCACGGAAGTTTTCAGAGATGAGAATGTGCCTTCGGGAACCGTGAGACAGGTGCTGCATGGCT
GTCGTCAGCTCGTGTTGTGAAATGTTGGGTTAAGTCCCGCAACGAGCGCAACCCTTATCCTTTGTTGCCA
GCGGTCCGGCCGGGAACTCAAAGGAGACTGCCAGTGATAAACTGGAGGAAGGTGGGGATGACGTCAAGTC
ATCATGGCCCTTACGACCAGGGCTACACACGTGCTACAATGGCGCATACAAAGAGAAGCGACCTCGCGAG
AGCAAGCGGACCTCATAAAGTGCGTCGTAGTCCGGATTGGAGTCTGCAACTCGACTCCATGAAGTCGGAA
TCGCTAGTAATCGTGGATCAGAATGCCACGGTGAATACGTTCCCGGGCCTTGTACACACCGCCCGTCACA
CCATGGGAGTGGGTTGCAAAAGAAGTAGGTAGCTTAACTTCGGGAGGGCG
```

Topological "Checksum"

## TISA unique ID

$$\begin{pmatrix} 174 & 0 & 0 & 146 \\ 0 & 233 & 180 & 0 \\ 0 & 143 & 154 & 0 \\ 185 & 0 & 0 & 230 \end{pmatrix}$$

Figure 10

*Step 1* - **Define windows of study**

**Figure 11**

## Random sequence #1

```
T T G T T T G A T G A T T T A T C
T T A G T A A A T A G T T G T A A
A C T G T A G A G T A A T G A A T
T G A T T T C C A A T G A A T T A
A A T G T G T G T T T A G C T T C
G T A T T G G T T A C G A T T A A
A A A A T T T T A A T C A A A A G
T A A A A A T T C G T A T T G T T
A T T T A G T T T A A A G A C T A
C T G A T A G T A T C T T G A T T
G T G T T A T C A T T A C T T T G
A G G A G C G C G G G T A A G T A
T A G A T A C G T G T T T T T A A
A A G G T T T G T A G A C T A A G
T A A A T C A A T A T T A A T G T
C A A T G T G T T T T T T C A A G
T T A G A A G T G G A T T C T T T
```

## Random sequence #2

```
G T T G A T T A T A G G A T T T A
T T A T A T T A T T G T C A A T G
G T T T A A T G T A C A T A G G G
A T C A G G T A A A T A T A T A A
A T T T T A A T T T A A T A T G A
A A C T T G T A T A A T A A T G T
T A T C T A A A A A G T G C G G T
A A A T T T C T G T A T T T A G C
T T A A A G A A A A A T T T C T A
T T A T T A G T A A T G A T T T G
T T A G T G G T C T A A T G T C T
A A T A A A A G A A T T A T A T A
A A T T G A G T A T T G T G C A T
T T T T T A A A T T A G G T T A A
G T A G T A T C C A G A A A G T G
T T C G A A C C A A A A A T C T A
A A T T T A C A T A A A A T T T
```

Step 2

Step 5

Step 5

# Figure 12

100

110

140

120

150

130

# Figure 13

Europäisches
Patentamt

European
Patent Office

Office européen
des brevets

**EUROPEAN SEARCH REPORT**

Application Number

EP 22 17 0387

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Chang Hsuan-T ET AL: "DNA Sequence Representation and Comparison Based on Quaternion Number System", (IJACSA) International Journal of Advanced Computer Science and Applications, 1 January 2012 (2012-01-01), pages 1-8, XP055964774, Retrieved from the Internet: URL:https://thesai.org/Downloads/Volume3No11/Paper_7-DNA_Sequence_Representation_and_Comparison_Based_on_Quaternion_Number_System.pdf [retrieved on 2022-09-26] | 1-9 | INV. G16B50/00 |
| A | * page 40, Cahpter II.A (Quaternion Number Sequence Representation) * ----- | 10-13 | |
| X | BASSI SEBASTIAN ET AL: "New checksum functions for Biopython", NATURE PRECEDINGS, 28 June 2007 (2007-06-28), XP055965138, DOI: 10.1038/npre.2007.278.1 Retrieved from the Internet: URL:http://www.nature.com/articles/npre.2007.278.1> * pages 2,3 * ----- | 1-13 | |
| X | SILVERMAN B.D. ET AL: "A measure of DNA periodicity", JOURNAL OF THEORETICAL BIOLOGY., vol. 118, no. 3, 7 February 1986 (1986-02-07), pages 295-300, XP055964596, GB ISSN: 0022-5193, DOI: 10.1016/S0022-5193(86)80060-1 | 1-9 | TECHNICAL FIELDS SEARCHED (IPC) G16B |
| A | * page 296, paragraph 1; figure 2 * ----- | 10-13 | |
| | -/-- | | |

The present search report has been drawn up for all claims

| Place of search | Date of completion of the search | Examiner |
|---|---|---|
| Munich | 6 February 2023 | Schmidt, Karsten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt
European
Patent Office
Office européen
des brevets

# EUROPEAN SEARCH REPORT

Application Number

EP 22 17 0387

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MAHMOOD AKHTAR ET AL: "On DNA Numerical Representations for Period-3 Based Exon Prediction", GENOMIC SIGNAL PROCESSING AND STATISTICS, 2007. GENSIPS 2007. IEEE INT ERNATIONAL WORKSHOP ON, IEEE, PI, 1 June 2007 (2007-06-01), pages 1-4, XP031152838, ISBN: 978-1-4244-0998-3 | 1-9 | |
| A | * page 2/4, Chapter "2.1.5 Quaternion" *<br>----- | 10-13 | |

TECHNICAL FIELDS
SEARCHED     (IPC)

The present search report has been drawn up for all claims

| Place of search | Date of completion of the search | Examiner |
|---|---|---|
| Munich | 6 February 2023 | Schmidt, Karsten |

EPO FORM 1503 03.82 (P04C01)

page 2 of 2